(19) Europäisches Patentamt
European Patent Office
Office européen des brevets

(11) Publication number: **0 644 260 A1**

## (12) EUROPEAN PATENT APPLICATION
## published in accordance with Art. 158(3) EPC

(21) Application number: 94902099.4

(22) Date of filing: **09.12.93**

(86) International application number:
**PCT/JP93/01791**

(87) International publication number:
**WO 94/13792 (23.06.94 94/14)**

(51) Int. Cl.6: **C12N 9/28**, C12N 1/20, C12P 19/14

(30) Priority: **09.12.92 JP 329108/92**

(43) Date of publication of application:
**22.03.95 Bulletin 95/12**

(84) Designated Contracting States:
**DE DK FR GB IT NL**

(71) Applicant: **TAKASAKI, Yoshiyuki**
**TIP 806,**
**5-30, Nishi 1-chome,**
**Tachibanadori,**
**Miyazaki-shi**
**Miyazaki-ken 880 (JP)**

(72) Inventor: **TAKASAKI, Yoshiyuki**
**TIP 806,**
**5-30, Nishi 1-chome,**
**Tachibanadori,**
**Miyazaki-shi**
**Miyazaki-ken 880 (JP)**

(74) Representative: **Boeters, Hans D. Dr. et al**
**Boeters & Bauer**
**Bereiteranger 15**
**D-81541 München (DE)**

(54) **NOVEL ACID- AND HEAT-RESISTANT -g(a)-AMYLASE, PROCESS FOR PRODUCING THE SAME, AND METHOD OF LIOUEFYING STARCH BY USING THE SAME.**

(57) A novel α-amylase excellent in acid and heat resistances, which is produced by a microorganism of the genus *Bacillus licheniformis* α and is capable of liquefying corn starch under an acidic condition of pH 4.0-5.5 at a temperature of 90-110 °C. Furthermore glucose can be obtained in a high yield by saccharifying corn starch with glycoamylase without adjusting the pH value.

Fig. 2

Field of the Invention

The present invention relates to a novel α-amylase, more particularly to a novel α-amylase having excellent resistance to acids and heat and which is capable of liquefying corn starch under acidic conditions at a pH 4.0 to 5.5 and a temperature of 90° to 110°C. The invention further relates to a process for preparing the α-amylase, and to a method for decomposing starch using this enzyme.

Background of the Invention

In the preparation of glucose oligosaccharides, from starch, the starch first is liquefied with a liquefying enzyme (α-amylase) produced by a microorganism belonging to the genus *Bacillus*, and then saccharified with glucoamylase produced by a mold belonging to the genus *Aspergillus* or *Rhizopus*. Starches from sources which grow underground, such as starches from potatoes or sweet potatoes, can be liquefied with comparative ease. Corn starch, which is a major starch source at the present time, is more difficult to liquefy. Corn starch can be liquefied by a two-stage liquefaction method, in which the starch first is partially liquefied and caused to swell by reacting it for a short period of time at a high temperature of about 135°C, and then further liquefied at about 90°C, after the addition of α-amylase. Another method, which is currently more popular, comprises treating corn starch with a heat resistant α-amylase produced by a microorganism of *Bacillus stearothermophilus* (K. Ogawa, A. Imanishi, T. Isemura, J. Biochem., 67, 65-75 (1970), S. Endoh, J. Fermentation Technology, 37, 353, 356 (1959)), *Bacillus licheniformis* (F.J. Morgan, et al, J. Applied Bacteriology, 50, 107-114 (1981), or R.M. Antrim, et al., Starch, 43, 355 (1991)), or *Bacillus subtilis* (Japanese Patent Laid-open (kokai) No. 34117/1983) using an apparatus called a jet cooker at pH 6 to 6.5 and a temperature of 103° to 110°C (residence time: about 5 minutes), injecting the treated starch, and holding it at 90° to 95°C for 1.5 to 2 hours to perform the liquefaction.

Almost all α-amylases heretofore known have exhibited poor acid resistance and have optimum activity at pHs between 6 and 9. Therefore, starch liquefaction typically has been carried out at a pH in the range of 6 to 6.5. However, the glucoamylase produced by *Aspergillus niger*, which is used for saccharification of the liquefied starch in the manufacture of glucose, has an optimum activity at a pH of about 4.5 and an optimum temperature of 60°C. Thus, to reconcile these conditions, a complicated process must be used which comprises liquefying the starch at pH 6 to 6.5, adding an acid to lower the pH to around 4.5, and then carrying out the saccharification reaction.

In addition, liquefaction at a pH above 6 induces alkaline isomerization, by which the reducing ends of the produced dextrin are isomerized to fructose, This results in the two glucose residues at the reducing ends remaining as maltulose (a compound bound by the α-1,4-linkage of glucose and fructose), when the dextrin is saccharified with glucoamylase (J.K. Shetty, W.G. Allen, Cereal Foods World, 33, 929 (1988)).

In view of these drawbacks, a number of researchers have attempted to accomplish the liquefaction of starch under acidic conditions at pH 6 or lower. For example, K. Goto, et al. reported that a mutant producing an acid resistant α-amylase, which is active at pH 5.5 and 105°C, was obtained by treating an α-amylase-producing microorganism *Bacillus licheniformis* IFO 12196, which is active at pH 6.5 and 105°C, with nitrosoguanidine in the presence of 35% corn starch (Agricultural Chemical Society of Japan, Summary of 1986 General Meeting, page 653). J.F. Shetty et al. presented a report on an α-amylase produced by *Bacillus licheniformis* L-170 (Taka-Therm II), which can suppress the production of maltulose at liquefaction of corn starch at pH 5.8, even though its optimum pH is 6.5 (Cereal Foods World, 33, 929 (1988)). R.L. Antrim et al. reported starch liquefaction at pH 5.5 using an α-amylase produced by a *Bacillus lichenifor‒mis* which has an optimum pH range of 5.5 to 6.0 (Starch, 43, 355 (1991)). Neilsen et al. reported that a heat resistant cyclodextrin producing enzyme (cyclodextrin-glucano transferase) produced by *Ther‒moanacrobactor* is capable of liquefying corn starch at pH 4.5 and 105°C (Food Technology, January, 102 (1991)). Also, as shown in Table 1, some kinds of microorganisms belonging to the genus *Clostridium* are known to produce an acid resistant α-amylase (Japanese Patent Laid-open (kokai) Nos. 115491/1986, 185185/1986). These enzymes, however, possess poor heat resistance.

As discussed above, although various studies have been undertaken concerning liquefaction of starches under acidic conditions, all α-amylase which have been heretofore found have exhibited insufficient resistance to both acid and heat Thus, it has not been possible to liquefy corn starch under commercially favorable conditions, e.g., at a temperature 103° to 105°C, and a pH of 5 or less, using a jet cooker.

2

Disclosure of Invention

The present inventors have undertaken an extensive search to find an α-amylase enzyme from natural sources having resistance to acid conditions and heat, which is capable of liquefying corn starch in the same pH range as that in which the liquefied starch is saccharified with glucoamylase (e.g., about pH 4.5). As a result, they have discovered a microorganism belonging to the genus *Bacillus* which produces an α-amylase exhibiting excellent resistance to acid conditions and heat. This α-amylase exhibits optimum activity at a pH of about 5 (pH 4.7 to 5.3 when reacted in the presence of 1% soluble starch at 80°C for 30 minutes),and at a temperature of 90°C (when reacted in the presence of 1% soluble starch for 10 minutes). The optimum temperature rises to about 100°C when reacted in the presence of 5 mM calcium chloride. The enzyme of the present invention is capable of liquefying corn starch under acidic conditions at pH 4.5 to 5.5 and at a temperature of 105° to 110°C in the presence of 30 to 35% corn starch.

Accordingly, an object of the present invention is to provide a novel α-amylase capable of liquefying corn starch under acidic conditions at pH 4.0 to 5.5 and a temperature of 100° to 110°C.

Another object of the present invention is to provide a process for producing an α-amylase having excellent resistance to both acid conditions and heat, the process comprising culturing a microorganism belonging to the genus *Bacillus licheniformis* capable of producing an α-amylase having excellent resistance to acids and heat, and collecting the α-amylase.

Still another object of the present invention is to provide a process for liquefying starch using said α-amylase.

The α-amylase having excellent resistance to acids and heat of the present invention can be obtained by culturing a microorganism belonging, for example, to the genus *Bacillus licheniformis*. This microorganism produces the enzyme into the culture medium, from which it can be isolated and purified.

The physicochemical characteristics of the acid and heat resistant α-amylase of the present invention are as follows:

(a) Activity and substrate specificity:

The present α-amylase enzyme catalyzes the endo-type decomposition of the α-1,4-glucosidic bond of starch and converts it to dextrin. Due to this activity, the starch viscosity rapidly decreases and the starch becomes liquefied, producing $G_5$ (maltopentaose consisting of five glucose molecules) and $G_6$ - (maltohexaose consisting of six glucose molecules) at the initial stage of the reaction, and ultimately producing a series of reducing oligosaccharides, such as glucose, maltose, maltotriose, maltotetraose, maltopentaose, and maltohexaose. This enzyme cannot hydrolyze the α-1,6-glucosidic bond of amylopectin or its derivatives.

(b) Active pH and optimum pH:

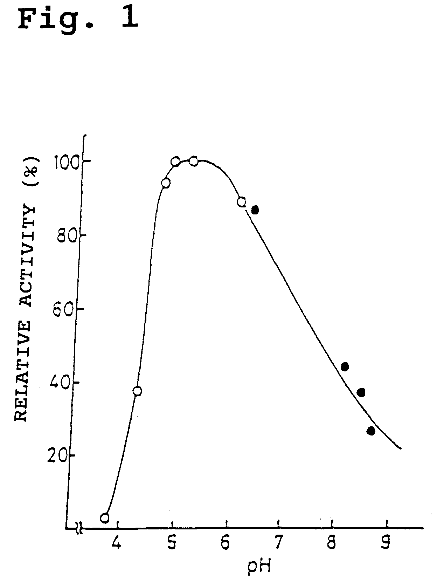

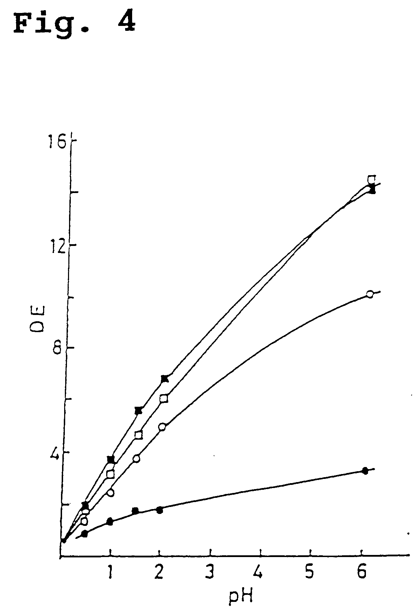

The enzyme is active within a wide pH range, i.e., pH 3.5 to 10. As shown in Table 3 for Example 2 hereinafter, optimum activity occurs in a pH range of 4.7 to 5.3, when the reaction is carried out at 80°C for 30 minutes in the presence of a 5 mM acetate buffer solution and 1% soluble starch. Excellent activity is exhibited even under acidic conditions of pH 5.5 or lower (see; Figure 1, —O— in acetate buffer, —●— in phosphate buffer). As is clear from Example 5 hereinafter, optimum activity was found to occur at about pH 5, when starch was subjected to primary liquefaction at 105°C for 5 minutes and secondary liquefaction at 95°C for 0.5 to 6 hours, using a 35% corn starch solution in 8 mM acetate buffer solution and in the presence of 5 mM $CaCl_2$, which are typical liquefaction conditions of corn starch (see; Figure 4, —□— at pH 5.25, —■— at pH 5.0, —O— at pH 4.75, —●— at pH 4.5). The enzyme exhibits good activity even at a pH of around 4.5 and can completely liquefy corn starch.

(c) Active temperature and optimum temperature

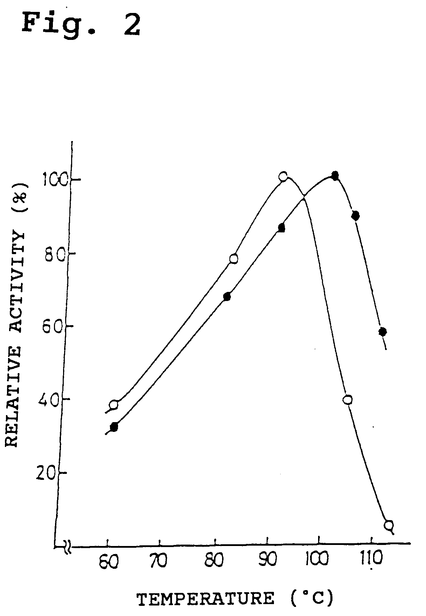

The enzyme is active up to a temperature of about 120°C, and has an optimum activity at a temperature of 90°C to 100°C. As shown in Table 4 (Example 3) hereinafter, the optimum temperature was found to be about 90°C, when the reaction was carried out using 1% soluble starch as a substrate in a 10 mM acetate buffer solution (pH 5.5) for 10 minutes (see; Figure 2 —O—); and about 100°C when 5 mM $CaCl_2$ was present (see; Figure 2 —●—). At a 30% starch concentration, which is a practical starch liquefaction condition, the enzyme can liquefy the starch at pH 4.0 to 5.5 and a temperature of 90°C to 110°C in the presence of calcium ions (Table 6).

(d) pH Stability

The activity of the enzyme is maintained at pH levels of about 5 to 10, when treated at 50°C for one hour. 80% or more of the activity can be maintained at a pH of about 4.5 to 11.

(e) Heat stability:

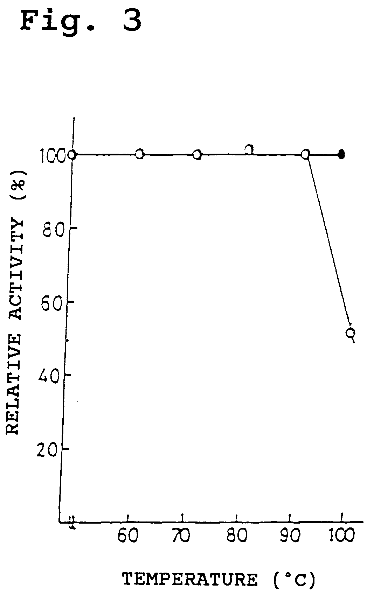

No inactivation of the enzyme is observed up to a temperature of 90°C, when heated for 10 minutes at 60° to 100°C in the presence of 5 mM acetate buffer solution (pH 5.5) (see; Figure 3 —O—). As

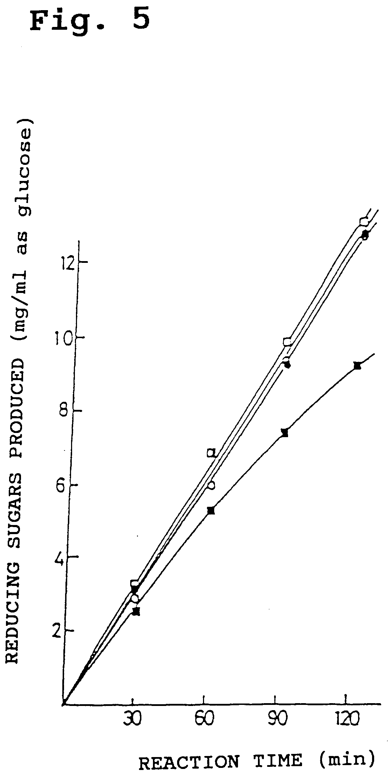

shown in Table 5 (Example 4), about 50% of the enzyme activity is lost when heated at 100°C for 10 minutes, however the enzyme is stable under these conditions in the presence of the substrate and $CaCl_2$. For example, in the presence of 5 mM $CaCl_2$, no inactivation was observed when the enzyme was heated for 10 minutes at 100°C (see; Figure 3 —●—), and almost no inactivation was found even after 30 minutes at this temperature. Under conditions of starch liquefaction, i.e., at a starch concentration of 30 to 35% and in the presence of $CaCl_2$, almost no inactivation of the enzyme was observed after heating for two hours at 100°C (see; Figure 5).

(f) Molecular weight:

The molecular weight as measured by gel filtration chromatography using Sephadex™ G-75 was about 9,000. This enzyme goes through an filtration membrane (YM-10™ manufactured by Amicon Co.) which does not allow compounds having a molecular weight of 10,000 or greater to pass and is blocked by an filtration membrane (YM-3™ manufactured by Amicon Co.) which does not allow compounds having a molecular weight of 3,000 or smaller to pass.

(g) Stability:

The enzyme is protected from inactivation by heat in the presence of $Ca^{2+}$, $Sr^{2+}$, $Ba^{2+}$, and $Na^+$ ions.

(h) Inhibition:

The activity of the enzyme is inhibited by $Zn^{2+}$, $Hg^{2+}$, $Ag^{2+}$, $Cu^{2+}$, $Fe^{2+}$, and $Al^{3+}$ ions.

(i) Purification method:

The enzyme can be purified from a supernatant obtained from a culture of $\alpha$-amylase producing cells by precipitation with 80% ammonium sulfate, DEAE-Sepharose column chromatography, Sephadex™ G-75 column chromatography, or the like under conditions such that the enzyme exhibits uniformity in electrophoresis.

The activity of the $\alpha$-amylase of the present invention was measured by the following methods:

(a) Iodine-staining method (dextrination titer):

An appropriate amount of a solution of the enzyme is added to 0.5 ml of a solution of 1% soluble starch dissolved in 0.1 M acetate buffer solution (pH 5.0). After the addition of distilled water to bring the total volume to 1.0 ml, the enzyme is reacted at 90°C. After 15 minutes, the reaction is terminated and, at the same time, the mixture is colored by adding 5 ml of an iodine solution (a mixture of 2.5 ml of an iodide liquid (0.2 g $I_2$ + 2 g KI) and 10 ml of 0.1 M HCl which was brought to 100 ml with distilled water). After allowing the mixture to stand for 15 minutes, a colorimetric measurement is carried out at 660 nm. One unit of activity was determined to be the amount of the enzyme decreasing 1% of the blue color in one minute.

(b) Viscosity method (dextrination titer):

The measurement is carried out conforming to JIS7001-1990. Specifically, 1 ml of the enzyme is added to 10 ml of a 0.1 M acetate buffer solution (pH 5.0) containing 1 g of potato starch and reacted at 65°C. One unit of activity is determined to be the amount of the enzyme required to make the viscosity of the solution the same as that of a control solution of silicone oil (dimethylpolysiloxane).

(c) Reducing sugar quantitative measurement method (saccharification titer):

An appropriate amount of the enzyme solution is added to 0.5 ml of a solution of 1% soluble starch dissolved in 0.1 M acetate buffer solution (pH 5.0). After the addition of sufficient distilled water to bring the total volume to 1.0 ml, the enzyme is reacted at 90°C. The reducing sugars produced are measured by the Somogyi-Nelson method. One unit of activity is determined to be the amount of enzyme producing reducing sugars corresponding to 1 $\mu$mol of glucose.

Tables 1 and 2 show a comparison of the characteristics of the $\alpha$-amylase of the present invention and known $\alpha$-amylases.

TABLE 1

Comparison of various heat resistant α-amylases. (1)

| Microorganism | Enzyme of the present invention | Enzyme produced by B. stearothermophilus | Enzyme produced by B licheniformis | Enzyme produced by B. subtilis MN-3851 | Enzyme produced by B. subtis |
|---|---|---|---|---|---|
| Optimum pH | About 4.7-5.3 (in 1% starch, 80°C, 30 min) | 5-6 [1] (60°C, 10 min) 4.6-5.1 (65°C, 10 min) | 7-9 [1] <br> 5-6 [2] (50°C, 1 mM $Ca^{2+}$) | 6-7 (in 1% starch, 40°C, 90 min) | 6-8 |
| Stable pH | 5-10 (50°C, 1 hr) | 6-11 (26°C, 30 min) | 7-11 [1] <br> 6-11 [2] (25°C, 30 min) | 5-11 (50°C, 1 hr) | 5-11 |
| Optimum temperature | About 90°C (in 1% starch, pH 5, 10 min) About 100°C (in $Ca^{2+}$) | 65-73°C [1] (10 min) 55-70°C | 90°C [1] <br> 76°C [2] (pH 9.0, 10 min) | 95-98°C (pH 6, in $Ca^{2+}$) | 70°C (in 1.3% starch) 85-90°C (in 35% starch, $Ca^{2+}$) |
| Heat stability | Not inactivated at pH 5.5, 90°C, 10 min; About 50% inactivated at 100°C in 10 min.; Not inactivated at 100°C in 30 min. in $Ca^{2+}$ | 17% inactivated at pH 6, 90°C in 6 min. [1] 29% inactivated at 85°C in 20 hrs. [2] | Stable at pH 8.0, below 60°C [2] | Almost no inactivation at pH 8, 90°C, 30 min. in $Ca^{2+}$ and starch; About 85% inactivated at pH 8, 80°C in 30 min. without $Ca^{2+}$ | Stable at 70°C or lower |
| Stability | $Ca^{2+}$, $Na^{2+}$ | $Ca^{2+}$ | $Ca^{2+}$ | $Ca^{2+}$ | $Ca^{2+}$, $Na^{+}$ |
| Inhibitor | $Cu^{2+}$, $Hg^{2+}$, $Ag^{+}$, $Zn^{2+}$, $Fe^{2+}$, $Al^{3+}$ | | | $Cu^{2+}$, $Hg^{2+}$, $Zn^{2+}$ | EDTA, $Cu^{2+}$, $Ag^{+}$, $Hg^{2+}$, $Pb^{2+}$ |
| Molecular weight | About 9,000 [a] | 48,000 [1] [b] | 62,500 [1] <br> 22,500 [2] | 30,000 [c] | 49,000 |
| Sources | | 1) K. Ogasawara J. Biol. Chem. 67, 65 (1970) 2) J. Biochem. 236, 2952 (1961) | 1) F. J. Morgan J. App. Bact., 50, 107 (1981) 2) N. Saito, Arch. Biochem. Biophy., 155, 290, (1973) | Fumio Hattori, JPA 34117/1983 | J. Biochem 67, 65 (1970) |

Measurement method: a) Gel permeation, b) Precipitation, c) Acrylamide electrophoresis

EP 0 644 260 A1

TABLE 2

Comparison of various heat resistant α-amylases (2)

| Microorganism | Enzyme produced by B. amyloliquefaciens | Enzyme produced by Thermophile V-2 | Enzyme produced by B. acidocaldarius | Enzyme produced by Clostridium sp. |
|---|---|---|---|---|
| Optimum pH | 6-7 | | 3.5 | 5.5 (80°C) 1) About 4.5 (60°C) 2) |
| Stable pH | 9.2 | | 4-5.5 | 2-7 2) (60°C, 30 min) |
| Heat stability | | | | 80°C, 30 min., 70% remained 1) (1 mM $Ca^{2+}$) |
| Optimum temperature (In 35% starch) | 80°C | 70°C | 70°C | 80°C (pH 4, $Ca^{2+}$) 1) 90°C (pH 6, $Ca^{2+}$) 2) |
| Stability | $Ca^{2+}$ | $Ca^{2+}$ | $Ca^{2+}$ | $Ca^{2+}$ |
| Inhibitor | | | | $Ni^{2+}$, $Cu^{2+}$, $Zn^{2+}$, $Mn^{2+}$ 1) $Ni^{2+}$, $Co^{2+}$, $Zn^{2+}$ 2) |
| Molecular weight | 50,000 | | 66,000 | 72,000±3,000 c) Above 20,000 2) d) |
| Reference | T. Kotaka, J. Jap. Soc. Starch Sci. 27, 151 (1980) | A. Hasegawa, J. Biochem., 29, 35 (1976) | M. Kanno, Agric. Biol. Chem., 51, 23 (1986) | 1) JPA 185185/1986 2) JPA 115491/1986 |

Measurement method: c) SDS acrylamide electrophoresis, d) Molecular siev

As clearly shown in Tables 1 and 2, one of the characteristics of the enzyme of the present invention is its superior resistance to both acid conditions and heat compared to conventional α-amylases. The enzyme of the present invention exhibits optimum activity at temperatures of 95° to 100°C even under acidic conditions at about pH 5 in a practical starch concentration of 30 to 35% in the absence of calcium ions; In the presence of $Ca^{2+}$, it exhibits almost no decrease in activity when heated at 100°C for 10 minutes (Table 5, Figure 3). It exhibits almost no decrease in activity after a reaction of two hours at 90°C, 95°C, or 100°C under acidic conditions at pH 4.5 to 5.25 (Table 7, Figure 5). Accordingly, the use of the enzyme of the present invention can be used to liquefy corn starch at the high temperatures (100° to 110°C) required for corn starch liquefaction under acidic conditions of about pH 4.0 to 5.5. In contrast, almost all α-amylases are unstable at acidic conditions of pH 6 or less, and cannot be used to liquefy corn starch under these conditions.

Another characteristic of the enzyme of the present invention is its extremely small molecular weight as compared with conventionally known α-amylases. As shown in Tables 1 and 2, the molecular weights of α-amylases heretofore reported are in the range of 20,000 to 70,000, whereas the enzyme of the present invention can go through an filtration membrane (YM-10™ manufactured by Amicon Co.) which does not allow compounds having a molecular weight of 10,000 or greater to pass, although it is blocked by an filtration membrane (YM-3™ manufactured by Amicon Co.) which does not allow compounds having a molecular weight of 3,000 or smaller to pass. The molecular weight of the enzyme of the present invention was predicted to be about 9,000 Daltons as determined by the gel filtration chromatography using Sephadex™ G-75. The following standard proteins were used as controls:, ribonuclease (M.W 13,700), chymotrypsin (M.W. 35,000), ovalbumin (M.W. 45,000), and serum albumin (M.W. 67,000). These results further support the conclusion that the enzyme of the present invention is a novel enzyme having an extremely small molecular weight. This extremely small molecular weight is believed to contribute to its high resistance to acids and heat.

The use of the enzyme of the present invention allows corn starch to be liquefied at pH 5 or less and allows impurities contained in corn starch, such as proteins, to be completely separated from the resulting dextrin, thus ensuring the production of liquefied starch (dextrin) having excellent filterability. The dextrin thus obtained has a superior resistance to aging. Only the use of the enzyme of the present invention, which is considered to exert an enzyme activity subtly different from that of conventionally known $\alpha$-amylases, resulted in the production of dextrin having such excellent properties.

The present novel $\alpha$-amylase enzyme can be obtained from $\alpha$-amylase-producing bacteria. The microorganism which produces the present $\alpha$-amylase, designated *Bacillus licheniformis*-$\alpha$, was obtained according to the following procedure. *Bacillus licheniformis*-$\alpha$ was obtained from soil (depth: about 5cm) of a field at Kiyotake-cho, Miyazaki-gun, Miyazaki-ken, Japan. A small amount of the soil was suspended in a sterilized water and the supernatant was applied to a medium comprising a mixture of 2% soluble starch, 1% polypeptone, 0.3% $K_2HPO_4$, 0.1% $MgSO_4 \cdot 7H_2O$, and 2.5% agar. The strain *Bacillus licheniformis*-$\alpha$ was selected from about 8000 stocks of microorganisms grown at 30°C.

This microorganism has been deposited with the Fermentation Research Institute, Agency of Industrial Science and Technology, deposition No. 13286, FERM P-13286, and later transferred to deposit under the Budapest Treaty at the same Institute (FERM BP-4480). The mycological characteristics of this microorganism are as follows:

(1) Shape: rod
(2) Gram's staining: +
(3) Motility: +
(4) Spores:
    Sporangium: non-swelling
    Shape: oval
    Site: neutral to end
(5) Catalase: +
(6) Growth in anaeorbic conditions: +
(7) V-P reaction: +
(8) pH in V-P broth: 5.3
(9) Acid production from glucose: +
(10) Gas production from glucose: -
(11) Liquefaction of gelatin: +
(12) Starch hydrolysis: +
(13) Utilization of citric acid: +
(14) Utilization of propionate: +
(15) Yolk reaction: -
(16) Nitrate reduction: +
(17) Growth at pH 6.8 (in a nutrient broth): +
(18) Growth at pH 5.7: +
(19) Growth in the presence of 5% NaCl: +
(20) Growth in the presence of 7% NaCl: +
(21) Growth at 10°C: -
(22) Growth at 30°C: +
(23) Growth at 55°C: +
(24) Growth at 65°C: -

In the above description, + indicates that the characteristic is positive, and - denotes that the characteristic is negative.

Based on the above mycological characteristics, the microorganism of the present invention was classified to Bergey's Manual of Systematic Bacteriology, seventh and eighth editions. As a result, the microorganism was identified as an analog of *Bacillus licheniformis* and was named *Bacillus lichenifor-*

*mis-α.*

*Bacillus licheniformis* is known to produce an α-amylase having comparatively high heat stability and an optimum temperature of 70°C, and an α-amylase having high heat stability and an optimum temperature of 90°C, as shown in Table 1. The optimum pH of the α-amylase which has an optimum temperature of 90°C is 7 to 9, which differs from the enzyme of the present invention.

*Bacillus licheniformis-α* can be grown in culture media containing organic nitrogen sources, such as, for example, soybean cake, soybean protein, corn steep liquor, meat extract, peptone, milk casein, and yeast extract. Polypeptone, polypeptone-S yeast extract, soybean protein, and corn steep liquor are preferred nitrogen sources. In addition to these organic nitrogen sources, inorganic nitrogen sources, such as ammonium sulfate, ammonium chloride, urea, and ammonium nitrate, can be used, as needed. The media also may contain a carbon source. For example, starch and starch-derived substances, such as liquefied starch, soluble starch, or dextrin, can be used as carbon sources.

In addition to these nitrogen and carbon sources, the media may comprise phosphates, salts of magnesium, salts of sodium, salts of potassium, and the like, as supplemental raw materials. The addition of phosphate, magnesium ions, and manganese ions is particularly effective. For example, 0.05 to 0.3% $K_2HPO_4$, as a phosphate; 0.01 to 0.3% $MgSO_4 \cdot 7H_2O$, as a magnesium salt; and 0.01 to 0.1% $CaCl_2$, as a calcium salt, may be used.

The α-amylase of the present invention is secreted from cells of *Bacillus licheniformis-α* microorganism. After culturing, the enzyme is collected from the culture medium by filtration or centrifugation, for example, and then may be sterilized.

Because the α-amylase of the present invention has excellent acid resistance and heat resistance, it is particularly useful for liquefaction of starch at high temperature of e.g., 100° to 110°C. The present α-amylase can be used in the same manner as conventional heat resistant α-amylases produced by bacteria such as *Bacillus subtilis* or *Bacillus licheniformis*, which are currently used for industrial starch liquefaction. However, in contrast to these conventional α-amylases, the α-amylase of the present invention can be used even under acidic conditions e.g., pH 4.0 to 5.5. Because of this, a starch suspension can be liquefied either without adjustment of the pH or after adjustment of the pH to 4.0 to 5.5. The resulting liquefied starch then can be subjected to saccharification using a glucoamylase enzyme produced by a mold belonging to the genus *Aspergillus* without further adjustment of the pH. Specifically, liquefied starch having a DE (dextrose equivalent) of about 10, which is useful for saccharification, can be manufactured by adding an appropriate amount of the enzyme of the present invention to a corn starch suspension with a concentration of about 30 to 35%, treating the mixture at 103° to 105°C for about 5 minutes using a jet cooker, and further treating the product at 90° to 95°C for from about 0.5 hours to several hours.

During starch liquefaction, a calcium salt may be added. For example, calcium chloride at a concentration of about 1 to 10 mM can be added as a calcium salt. The anion component in the calcium salt greatly affects the enzyme stability when the liquefaction of starch is carried out at a temperature of 100°C or higher and at pH 5 or less. Calcium carbonate, quick lime, and the like also can be used as calcium sources.

## Brief description of drawing

Figure 1 shows the optimum pH of the α-amylase of the present invention. —○— when acetate buffer used, —●— when phosphate buffer ($KH_2PO_4$-$NaHPO_4$) used,

Figure 2 shows the optimum temperature of the α-amylase of the present invention. —○— in the absence of $CaCl_2$, —●— in the presence of 5 mM $CaCl_2$

Figure 3 shows the heat stability of the α-amylase of the present invention.

The suvival amounts of the activity were shown when heated at 90, 95 and 100°C at pH 5.5. —○— in no addition of $CaCl_2$, —●— in the addition of 5 mM $CaCl_2$

Figure 4 shows the results that the primary liquefaction of about 35% corn starch were carried out using the α-amylase of the present invention in 8 mM acetate buffer containing 5 mM $CaCl_2$ at 105°C for five minutes, following the secondary liquefactory at 90°C.

—●—, —○—, —□— and —■— show the results of the primary liquefactory in acetate buffer at pH 4.5, 4.7, 5.0 and 5.5, respectively

Figure 5 shows the results that the liquefaction of about 35% potato starch were carried out using the α-amylase of the present invention in 8 mM acetate buffer containing 5 mM $CaCl_2$.

—●—, —○—, —□— and —■— show the results of the primary liquefactory at temperature of at 90, 95 and 100°C, respectively.

Best Mode for Carring Out the Invention

The present invention is illustrated by the following examples.

Other features of the invention will become apparent in the following description of the exemplary embodiments which are provided for illustration of the invention and are not intended to be limiting thereof.

EXAMPLE

Example 1

200 ml of a medium (pH 5.5) comprising 2% soybean cake, 2% soluble starch, 0.05% $MgSO_4 \cdot 7H_2O$, 0.2% $K_2HPO_4$, and 0.05% $CaCl_2 \cdot 2H_2O$, was placed in a 1 liter conical flask, and sterilized at 121°C for 15 minutes. The medium was inoculated with *Bacillus licheniformis*-α (FERM BP-4480) and cultured at 30°C for 5 days, while rotating the flask at 225 rpm. As a result, 1.5 unit of the enzyme was produced per 1 ml of the medium.

The culture broth was centrifuged to obtain a supernatant liquid. Ammonium sulfate was added to the supernatant liquid to a concentration of 80% to induce precipitation. The filtrate was dialyzed and concentrated using an filtration membrane (YM-3™ manufactured by Amicon Co.) to obtain a 31.8 unit/ml (measured using the viscosity method) enzyme solution.

Example 2

The α-amylase of the present invention obtained in Example 1 was reacted with a solution of 1% soluble starch in a 0.1 M buffer solution for 30 minutes to examine the pH dependency of the α-amylase activity. An acetate buffer or a phosphate buffer ($KH_2PO_4$-$Na_2HPO_4$) was used as the buffer solution. The results are shown in Table 3.

TABLE 3

| pH | Relative activity (%) | Buffer solution |
|-----|-----|-----|
| 3.6 | 8.0 | Acetate buffer |
| 4.2 | 34.0 | Acetate buffer |
| 4.5 | 98.0 | Acetate buffer |
| 4.7 | 100 | Acetate buffer |
| 5.0 | 100 | Acetate buffer |
| 5.9 | 87.0 | Acetate buffer |
| 6.2 | 86.0 | Phosphate buffer |
| 8.1 | 44.0 | Phosphate buffer |
| 8.4 | 37.0 | Phosphate buffer |
| 8.7 | 27.0 | Phosphate buffer |

As can be seen from Table 3, the α-amylase of the present invention is active at pH 4 to 8.5, and the activity at pH 4.5 to 5.5 is extremely high.

Example 3

The α-amylase of the present invention obtained in Example 1 was reacted with a solution comprising 1% soluble starch in a 10 mM buffer solution for 10 minutes to examine the temperature dependency of the α-amylase activity in the case where no $CaCl_2$ was added, and the case where 5 mM $CaCl_2$ was added. The results are shown in Table 4 and Figure 2.

TABLE 4

| Relative activity (%) vs. temperatures | | |
|---|---|---|
| Temperature (°C) | No CaCl$_2$ was added | 5 mM CaCl$_2$ was added |
| 60 | 48.5 | 33 |
| 70 | 55 | 48 |
| 80 | 77 | 68 |
| 90 | 100 | 86 |
| 100 | 62 | 100 |
| 104 | 47 | 99 |
| 113 | 5 | 58 |

As can be seen from Table 4, the α-amylase of the present invention exhibits a high activity at 90°C in the absence of calcium ions. When calcium ions are present, it exhibits a high activity at 90° to 104°C.

Example 4

The α-amylase of the present invention obtained in Example 1 was heated at 60° to 100°C for 10 minutes in a 5 mM acetate buffer solution (pH 5.5) to examine the residual activity. The results are shown in Table 5 and Figure 3.

TABLE 5

| Heat stability test | | |
|---|---|---|
| Temperature (°C) | Relative activity (%) | |
| | No CaCl$_2$ was added | 5 mM CaCl$_2$ was added |
| 60 | 100 | 100 |
| 70 | 100 | 100 |
| 80 | 100 | 100 |
| 90 | 100 | 100 |
| 100 | 52 | 100 |

As can be seen from Table 5, the α-amylase of the present invention did not lose any activity by heating up to 90°C in the absence of calcium ions. When heated at 100°C, 50% of the activity was lost. When calcium ions were present, almost no deactivation was seen after in 30 minutes when heated at 100°C.

Example 5

Corn starch was liquefied using the α-amylase of the present invention obtained in Example 1. About 35% corn starch in a 8 mM acetate buffer solution was heated in the presence of 5 mM CaCl$_2$ at 105°C for 5 minutes (primary liquefaction) and at 90°C for 0.5 to 6.0 hours (secondary liquefaction). The results are shown in Table 6 and Figure 4.

TABLE 6

| pH | | Dextrose equivalent (%) | | | | |
|---|---|---|---|---|---|---|
| | | Reaction time (hours) | | | | |
| Initial | Final | 0.5 | 1.0 | 1.5 | 2.0 | 6.0 |
| 4.50 | 4.27 | 0.9 | 1.4 | 1.5 | 1.8 | 3.2 |
| 4.70 | 4.58 | 1.4 | 2.5 | 3.8 | 4.9 | 10.2 |
| 5.02 | 4.97 | 2.1 | 3.7 | 5.5 | 6.8 | 14.2 |
| 5.25 | 5.27 | 1.8 | 3.1 | 4.5 | 6.0 | 14.5 |

Example 6

Liquefaction conditions for corn starch were studied using the $\alpha$-amylase of the present invention obtained in Example 1.

1.6 unit (as determined by the viscosity method) of the $\alpha$-amylase obtained in Example 1 was added to a mixture of 0.4 g of potato starch, 0.2 ml of 0.4 M acetate buffer solution (pH 5.5), and 0.05 ml of 0.1 M $CaCl_2$. The total volume was brought to 1.0 ml with distilled water, and the mixture was reacted at 90°C, 95°C, 100°C and 105°C.

A specified amount of the reaction product was sampled at prescribed intervals to quantitatively measure the reducing sugars produced, as glucose, by the Somogyi-Nelson method. The results are shown in Table 7 and Figure 5.

TABLE 7

| Influence of temperature on starch liquefaction | | | | |
|---|---|---|---|---|
| Temperature (°C) | Reducing sugars produced (mg/ml as glucose) | | | |
| | 0.5 hr. | 1.0 hr. | 1.5 hrs. | 2.0 hrs. |
| 90 | 2.78 | 5.90 | 9.30 | 12.40 |
| 95 | 3.11 | 6.85 | 9.14 | 12.60 |
| 100 | 3.19 | 7.80 | 9.80 | 13.00 |
| 105 | 2.44 | 5.22 | 7.31 | 9.20 |

Example 7

In this Example, the influence of pH on starch liquefaction was studied.

To 3.2 unit of the $\alpha$-amylase obtained in Example 1, 0.4 g of potato starch, 0.2 ml of 0.4 M acetate buffer solution (pH 4.5 to 5.25), and 0.05 ml of 0.1 M $CaCl_2$ were added to make a total volume of 1.0 ml. The reaction was carried out at pHs 4.5, 4.75. 5.0, and 5.25, at 90°C for 30, 60, 90, and 120 minutes, for each pH. The reducing sugars produced were quantitatively measured, as glucose, by the Somogyi-Nelson method. The results are shown in Table 8.

TABLE 8

| Influence of pH on starch liquefaction (Reaction temperature 90°C) | | | | |
|---|---|---|---|---|
| Reaction time (min) | Reducing sugars produced (mg/ml as glucose) | | | |
| | pH 4.5 | pH 4.75 | pH 5.0 | pH 5.25 |
| 30 | 1.01 | 1.93 | 2.62 | 2.61 |
| 60 | 1.95 | 3.92 | 5.66 | 5.53 |
| 90 | 2.80 | 6.05 | 8.40 | 9.56 |
| 120 | 3.73 | 8.79 | 10.80 | 11.70 |

As can be seen from Table 8, an optimum pH was found to be approximately 5 for up to 60 minutes at 90°C using a high concentration of starch. No significant inactivation of the enzyme took place after a 120 minute reaction at all pHs.

Example 8

This example shows the results of a test for liquefying corn starch.

A mixture of 3.2 unit (determined by the viscosity method) of the $\alpha$-amylase of the present invention obtained in Example 1, 0.4 g of corn starch, 0.2 ml of 0.4 M acetate buffer solution (pH 4.5, 4.75, 5.0 and 5.25), and 0.1 ml of 0.1 M $CaCl_2$ was added to a test tube with a screw cap to make a total volume of 1.0 ml. The primary liquefaction was carried out with heating at 105°C for 5 minutes in an oil bath, followed by secondary liquefaction at temperatures of 90°C and 95°C, each for 6 hours. A specified amount of the reaction product was sampled at 0.5, 1.0, 1.5, 2.0 and 6 hours after the reaction to quantitatively measure the reducing sugars produced by the Somogyi-Nelson method and to determine the DE. The results are shown in Tables 9 and 10. Here, DE represents the Dextrose Equivalent, which is a percentage of the reducing sugars (glucose) in the solid components.

TABLE 9

| Starch liquefaction (1) | | | | | |
|---|---|---|---|---|---|
| Primary Reaction (105°C) | Secondary Reaction (95°C) | Dextrose equivalent (%) pH | | | |
| | | pH 4.50 | pH 4.70 | pH 5.02 | pH 5.25 |
| 5 (minutes) | 0 (hour) | 0.48 | 0.65 | 0.94 | 0.73 |
| | 0.5 | 0.91 | 1.38 | 1.95 | 1.72 |
| | 1.0 | 1.37 | 2.45 | 3.64 | 3.07 |
| | 1.5 | 1.52 | 3.76 | 5.48 | 4.62 |
| | 2.0 | 1.76 | 4.92 | 6.73 | 6.01 |
| | 6.0 | 3.23 | 10.20 | 14.20 | 14.5 |
| pH after two hour reaction | | 4.27 | 4.58 | 4.97 | 5.27 |

TABLE 10

| Starch liquefaction (2) | | | | | |
|---|---|---|---|---|---|
| Primary Reaction (105°C) | Secondary Reaction (90°C) | Dextrose equivalent (%) pH | | | |
| | | pH 4.50 | pH 4.70 | pH 5.02 | pH 5.25 |
| 5 (minutes) | 0.5 (hour) | 0.57 | 1.34 | 2.20 | 2.41 |
| | 1.0 | 1.15 | 2.40 | 3.72 | 4.29 |
| | 1.5 | 1.67 | 3.60 | 4.97 | 5.95 |
| | 2.0 | 2.25 | 4.46 | 6.88 | 7.17 |
| | 6.0 | 6.40 | 11.30 | 13.88 | 17.47 |
| pH after two hour reaction | | 4.49 | 4.77 | 4.97 | 5.27 |

As can be seen from Tables 9 and 10, the enzyme of the present invention could effectively liquefy starch even under acidic conditions at pH 4.5 to 5 using the same liquefaction conditions as used for conventional α-amylases (primary liquefaction: 105°C, 5 minutes; secondary liquefaction: 90°C to 95°C).

Example 9

Experiments were carried out on the liquefaction of corn starch by changing the amount of enzyme.

The enzyme prepared in Example 1 was added in the amounts shown in Table 11 to a mixture of 0.4 g of corn starch, 0.2 ml of 0.4 M acetate buffer solution (pH 4.5 or 4.75), and 0.1 ml of 0.1 M $CaCl_2$ to make a total volume of 1.0 ml. Primary liquefaction was carried out with heating at 105°C for 5 minutes, followed by secondary liquefaction at 90°C for 6 hours. A specified amount of the reaction product was sampled at a prescribed interval to quantitatively measure the sugars produced by the Somogyi-Nelson method and to determine the DE. The results are shown in Table 11.

TABLE 11

| Starch liquefaction (3) | | | | | | |
|---|---|---|---|---|---|---|
| Reaction time (hour) | at pH 4.5 | | | | at pH 4.75 | |
| | 0.9 U | 13.5 U | 18.0 U | 22.5 U | 8.0 U | 16.0 U |
| 0.5 | 2.3 | 2.7 | 3.3 | 3.8 | 1.3 | 3.1 |
| 1.0 | 2.8 | 3.3 | 4.1 | 4.9 | 2.4 | 5.5 |
| 2.0 | 3.7 | 4.8 | 6.7 | 7.7 | 4.5 | 9.0 |
| 6.0 | 7.5 | 10.6 | 13.0 | 14.4 | 11.3 | 17.5 |

As clearly shown in Table 11, the DE increased as the amount of enzyme increased. Liquefied starch having a DE of about 10, a required DE for saccharification using glucoamylase, were obtained even under acidic conditions at pH 4.5 or 4.75. The liquefied products contained a scum of impurities, such as proteins contained in corn starch, which were filtered out very easily. The dextrin thus obtained exhibited excellent age stability.

Example 10

The liquefied corn starch (DE 18) obtained in Example 8 at pH 4.5 was saccharified using glucoamylase produced by *Aspergillus niger* (Novo Industry, Denmark).

The reaction was carried out at 60°C for 25 hours using the liquefied starch (about 30%), 8 mM acetate buffer solution (pH 4.5), and glucoamylase (6.1 unit/g substrate), and in the absence or presence of pullulanase (0.5 unit/g substrate). The sugar composition of the saccharified solution was analyzed by high performance liquid chromatography. The results are shown in Table 12.

TABLE 12

| Sugar composition of a saccharified liquid of corn starch liquefied at pH 4.5 | | | | | | | | | |
|---|---|---|---|---|---|---|---|---|---|
| Enzyme | 16 Hours | | | 20 Hours | | | 25 Hours | | |
| | $G_1$ | $G_2$ | $G_3-$ | $G_1$ | $G_2$ | $G_3-$ | $G_1$ | $G_2$ | $G_3-$ |
| Glucoamylase | 94.8 | 1.7 | 3.5 | 95.3 | 2.5 | 2.2 | 95.3 | 2.6 | 2.1 |
| Glucoamylase pullulanase | 97.5 | 1.6 | 0.9 | 97.4 | 1.8 | 0.8 | 97.4 | 1.8 | 1.1 |

Glucose was obtained at a high yield (about 95.3%), when saccharification was carried out using only glucoamylase. A high yield of about 97.5% of glucose also was achieved, when saccharification was carried out using glucoamylase in the presence of pullulanase. It was confirmed that there is a tendency for a saccharified solution containing a small content of $G_2$ (disaccharides) to be obtained when the saccharification is carried out using starch liquefied at a pH lower than 5.

One glucoamylase unit here indicates an amount of the enzyme producing 1 $\mu$mol of glucose per minute, when the reaction is carried out at pH 5.0 and at 40°C in a solution comprising 1% soluble starch (in a 5 mM acetate buffer solution). One pullulanase unit indicates an amount of the enzyme producing 1 $\mu$mol of glucose per minute, when the reaction is carried out at pH 5.0 and at 50°C in a solution comprising 1% pullulan (in a 5 mM acetate buffer solution).

As illustrated above, the $\alpha$-amylase produced by the microorganism belonging to the genus *Bacillus* of the present invention possesses excellent acid resistance and heat resistance, and can liquefy corn starch at 105°C and 90-95°C under acidic conditions of pH 5 or less. The liquefied product can be used as a raw material for glucoamylase-catalyzed saccharification without adjusting the pH. Furthermore, dextrin having excellent resistance to aging can be obtained using the $\alpha$-amylase of the present invention.

Industrial Applicability

The $\alpha$-amylase produced by the microorganism belonging to the genus *Bacillus* of the present invention possesses excellent acid resistance and heat resistance, and can liquefy corn starch at primary liquefaction at 105°C and secondary liquefaction at 90-95°C under acidic condition of pH 5 or less. The liquefied starch can be used as a raw material for glucoamylase-catalyzed saccharification without adjusting the pH.

REFERENCE OF MICROORGANISM

1 *Bachillus licheniformis* $\alpha$

Organization of Deposition:

National Institute of Bioscience and Human-Technology, Agency of Industrial Science and Technology, Ministry of International Trade and Industry

Address:

1-3, Higashi 1 chome, Tsukuba-shi, Ibaraki-ken, Japan

Deposition Number:

FERM BP-4480
(Transfer from FERM P-13286 on November 16, 1992)

**Claims**

1. An $\alpha$-amylase having the optimum pH of 5.5 or lower and the optimum temperature of above 90°C, produced by a microorganism belonging to the genus *Bacillus*.

2. The $\alpha$-amylase as defined in Claim 1, wherein said microorganism belonging to the genus *Bacillus* is a microorganism of *Bacillus licheniformis*.

3. The $\alpha$-amylase as defined in Claim 1, wherein said microorganism belonging to the genus *Bacillus* is *Bacillus licheniformis*-$\alpha$ (FERM BP-4480).

4. The $\alpha$-amylase as defined in Claim 1, possessing the following physicochemical characteristics:
   (1) Action and substrate specificity
   effects the endo-type decomposition of the $\alpha$-1,4-glucoside bond of starch and converts it to dextrin, and cannot hydrolyze the $\alpha$-1,6-glucosidic bond of amylopectin or its derivatives.
   (2) active pH and optimum pH
   pH at which the enzyme is active: 3.5 to 10
   optimum pH: 5 (measured at 80°C in a 5 mM acetate buffer solution and in the presence of 1% soluble starch.)
   (3) active temperature and optimum temperature
   maximum temperature at which the enzyme is active:
   120°C
   optimum temperature:
   90°C (when reacted using 1% soluble starch in a 5 mM acetate buffer solution (pH 5.5) for 10 minutes), or
   100°C (when reacted using 1% soluble starch in a 5 mM acetate buffer (pH 5.5) for 10 minutes in the presence of 5 mM $Ca^{2+}$);
   (4) pH Stability
   stable at pH 5 to 10 (when treated at 50°C for one hour in a 0.1 M acetate buffer solution or 0.1 M phosphate buffer solution);
   (5) heat stability
   stable at 90°C; 50% of the activity is lost when heated for 10 minutes at 100°C at pH 5.5 in a 5 mM acetate buffer solution; not deactivated when heated at 100°C for 10 minutes in the presence of $Ca^{2+}$;
   (6) molecular weight
   about 9,000 Daltons (as measured by gel filtration method);
   (7) stabilization
   protected from deactivation by heat in the presence of $Ca^{2+}$, $Sr^{2+}$, $Ba^{2+}$, and $Na^{+}$; and
   (8) inhibition
   activity is inhibited by $Zn^{2+}$, $Hg^{2+}$, $Ag^{2+}$, $Cu^{2+}$, $Fe^{2+}$, and $Al^{3+}$.

5. A microorganism belonging to the genus *Bacillus* capable of producing an $\alpha$-amylase having resistance to acids and heat as defined in Claim 1.

6. A microorganism defined in Claim 5 belonging to the species *Bacillus licheniformis*.

7. *Bacillus licheniformis*-$\alpha$ (FERM BP-4480)

8. A process for producing an $\alpha$-amylase having excellent resistance to acids and heat, comprising culturing a microorganism belonging to the genus *Bacillus licheniformis* capable of producing the $\alpha$-amylase defined in Claim 1, and collecting the $\alpha$-amylase from the culture broth.

9. A process for manufacturing dextrin which comprises reacting starch with the $\alpha$-amylase defined in Claim 1 at a temperature of 90°C or higher and at pH 5.5 or lower, and hydrolyzing the starch.

# Fig. 1

# Fig. 2

# Fig. 3

# Fig. 4

# Fig. 5

## INTERNATIONAL SEARCH REPORT

| International application No. |
|---|
| PCT/JP93/01791 |

**A. CLASSIFICATION OF SUBJECT MATTER**

Int. Cl$^5$ C12N9/28, C12N1/20, C12P19/14

According to International Patent Classification (IPC) or to both national classification and IPC

**B. FIELDS SEARCHED**

Minimum documentation searched (classification system followed by classification symbols)

Int. Cl$^5$ C12N9/28, C12N1/20, C12P19/14

Documentation searched other than minimum documentation to the extent that such documents are included in the fields searched

Electronic data base consulted during the international search (name of data base and, where practicable, search terms used)

WPI, WPI/L, BIOSIS PREVIEWS

**C. DOCUMENTS CONSIDERED TO BE RELEVANT**

| Category* | Citation of document, with indication, where appropriate, of the relevant passages | Relevant to claim No. |
|---|---|---|
| X | Journal of General and Applied Microbiology, Vol. 38, No. 4, August, 1992 (08. 1992) F. JIN et. al. "Purification and characterization of a thermostable α-amylase from Bacillus sp-JF strain" P. 293-302 | 1, 5, 8, 9 |
| X | Acta Microbiologica Sinica, Vol. 31, No. 4, (1991), Hu X et. al. "Studies on the | 1-3, 5-6, 8, 9 |
| A | screening of thermostable α-amylase-producing strains" P. 267-273 | 4, 7 |

☐ Further documents are listed in the continuation of Box C.   ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier document but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | |
| "O" | document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" document member of the same patent family |

| Date of the actual completion of the international search | Date of mailing of the international search report |
|---|---|
| March 8, 1994 (08. 03. 94) | March 22, 1994 (22. 03. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)